# EUROPÄISCHE PATENTANMELDUNG

(11) **EP 4 683 107 A1**
(43) Veröffentlichungstag der Anmeldung: **21.01.2026**
(21) Anmeldenummer: 24189133.2
(22) Anmeldetag: 17.07.2024
(51) Int. Cl.: H01P 5/18, A61B 6/00

(54) **SYMMETRISCHER RICHTKOPPLER BASIEREND AUF DIELEKTRISCHEN WELLENLEITERN**

(71) Anmelder: Siemens Healthineers AG, 91301 Forchheim (DE)
(72) Erfinder: Dr. Fackelmeier, Andreas, 91177 Thalmässing (DE)
(74) Vertreter: Siemens Healthineers Patent Attorneys

(57) **Zusammenfassung**

Es soll eine robuste kontaktlose Datenübertragung ermöglicht werden. Dazu wird eine Datenübertragungsvorrichtung (8) bereitgestellt, die einen ersten dielektrischen Wellenleiter (9) mit einem ersten Koppelabschnitt (23) und einen zweiten dielektrischen Wellenleiter (10) besitzt, der gegenüber dem ersten Koppelabschnitt (23) in einer Bewegungsrichtung bewegbar ist und einen ersten variablen Abstand (d1) zu dem ersten Koppelabschnitt (23) aufweist. Darüber hinaus besitzt die Datenübertragungsvorrichtung (8) einen dritten dielektrischen Wellenleiter (12), der einen zweiten Koppelabschnitt (25) und einen zweiten variablen Abstand (d2) zu dem zweiten dielektrischen Wellenleiter (10) aufweist. Der erste dielektrische Wellenleiter (9) ist fest in Bezug auf den dritten dielektrischen Wellenleiter (12) angeordnet. Der erste Koppelabschnitt (23) und der zweite Koppelabschnitt (25) sind in der Bewegungsrichtung gleich lang und liegen senkrecht zu der Bewegungsrichtung einander gegenüber.

## Beschreibung

Die vorliegende Erfindung betrifft eine Datenübertragungsvorrichtung zur kontaktlosen Datenübertragung, die einen ersten, zweiten und dritten dielektrischen Wellenleiter aufweist. Der erste dielektrische Wellenleiter besitzt einen ersten Koppelabschnitt und der dritte dielektrische Wellenleiter einen zweiten Koppelabschnitt. Der zweite dielektrische Wellenleiter ist gegenüber dem ersten Koppelabschnitt und dem zweiten Koppelabschnitt bewegbar.

Darüber hinaus betrifft die vorliegende Erfindung eine Bildgebungsvorrichtung sowie ein Verfahren zur kontaktlosen Datenübertragung.

Unabhängig vom grammatikalischen Geschlecht eines bestimmten Begriffes sind Personen mit männlicher, weiblicher oder anderer Geschlechteridentität mit umfasst.

Ein Computertomograph weist typischerweise eine feststehende Basis und eine sogenannte Gantry auf, wobei sich die Gantry im Betrieb, also während einer Datenerfassung mittels eines Röntgendetektors, relativ zur feststehenden Basis bewegt und hierbei eine Rotationsbewegung ausführt. Dabei ist der Röntgendetektor, mit dessen Hilfe die Generierung von Daten für eine Bilderzeugung erfolgt, üblicherweise Teil der Gantry, so dass die in Betrieb generierten Daten von der Gantry über eine Datenübertragungsstrecke hin zur feststehenden Basis und üblicherweise hin zu einer Aufbereitungseinheit für die generierten Daten übertragen werden müssen.

Die Übertragung der generierten Daten über die Datenübertragungsstrecke erfolgt dabei typischerweise ohne eine Zwischenspeicherung oder relevante Pufferung und dementsprechend quasi parallel zur Generierung von entsprechenden Daten und somit also auch während der Rotation der Gantry. Hierfür geeignete Datenübertragungsstrecken sind prinzipiell aus dem Stand der Technik bekannt, wobei in den meisten Fällen ein sogenannter Schleifring Teil einer entsprechenden Datenübertragungsstrecke ist.

Eine Ausführung einer für eine derartige Datenübertragung geeigneten Datenübertragungsstrecke ist in der DE 10 2016 208 539 A1 beschrieben. Diese umfasst einen dielektrischen Wellenleiter und ist auch für eine kontaktlose Datenübertragung geeignet.

Die immer höheren Datenmengen infolge der höheren Auflösung bei der Bildgebung und die daraus entstehenden Anforderungen an die Datenrate bei der Übertragung stellen ein noch nicht gelöstes Problem dar. Ein Ansatz ist, die Datenraten über den Einsatz von Richtkopplern, welche auf dielektrischen Wellenleitern basieren, zu verbessern. Die Übertragung erfolgt hierbei im Wesentlichen durch das elektromagnetische, insbesondere optische Einkoppeln eines Signals, welches durch einen dielektrischen Wellenleiter auf der Gantry geführt wird, in einen weiteren dielektrischen Wellenleiter am Halterahmen über den Luftspalt hinweg.

Ein gravierendes Problem ist hierbei ein nur schwer zu kontrollierender axial und radial variierender Versatz des Halterahmens zur Gantry während des Betriebs des CT infolge der Rotation der Gantry. Durch diesen variierenden Versatz ist der Abstand der beiden dielektrischen Wellenleiter, welche die wesentlichen Bestandteile des Richtkopplers bilden, nicht konstant. Aus der jeweiligen Ebene der beiden Wellenleiter betrachtet hat eine derartige Abstandsvariation eine normale und eine longitudinale Komponente, wobei die Variation der longitudinalen Komponente erhebliche Auswirkungen auf die Kopplungsstärke, also auf die empfangene Signalstärke aufweist. Die Kopplungsstärke zeigt dabei einen komplexen funktionalen Zusammenhang zwischen dem Abstand der beiden Wellenleiter zueinander sowie ihrer jeweiligen geometrischen Parameter (vorrangig: Kopplungslänge und Querschnitt).

Es wurde angedacht einen 60 GHz Verstärker sende- bzw. TX-seitig oder besser als LNA (low noise amplifier) empfangs- bzw. RX-seitig einzufügen, was sehr hohe Zusatzkosten verursachen würde. Ferner ist eine Optimierung der gesamten Übertragungsstrecke auf eine niedrigere maximale RF-Dämpfung, eine niedrige Dämpfungsvariation (Dynamik) und eine hohe mechanische Stabilität sehr aufwändig, was auch zu sehr hohen Kosten führen würde.

Das genannte Problem ist jedoch nicht nur auf den Bereich medizinischer Tomographen beschränkt, sondern generell von Relevanz, wenn in einem Gerät mit zwei Geräteteilen, welche im Betrieb des Geräts eine Relativbewegung zueinander durchführen, Daten oder auch Energie von einem an den anderen Geräteteil zu übertragen sind, und dies aufgrund der Relativbewegung über eine Kabelverbindung nicht oder nicht sinnvoll zu erreichen wäre.

Ausgehend hiervon liegt der Erfindung die Aufgabe zugrunde, eine vorteilhafte Lösung zur Datenübertragung anzugeben.

Diese Aufgabe wird erfindungsgemäß gelöst durch eine Datenübertragungsvorrichtung sowie durch eine Bildgebungsvorrichtung und ein Verfahren gemäß den unabhängigen Ansprüchen.

Bevorzugte Weiterbildungen sind in den rückbezogenen Ansprüchen enthalten. Die im Hinblick auf die Datenübertragungsvorrichtung angeführten Vorteile und bevorzugten Ausgestaltungen sind sinngemäß auch auf die Bildgebungsvorrichtung und das Verfahren übertragbar und umgekehrt.

Erfindungsgemäß wird demnach eine Datenübertragungsvorrichtung zur kontaktlosen Datenübertragung bereitgestellt. Die Datenübertragung erfolgt vorzugsweise im optischen Wellenlängenbereich (z. B. mit 60 GHz). Daher wird die Datenübertragungsvorrichtung mit dielektrischen Wellenleitern realisiert.

Die Datenübertragungsvorrichtung besitzt einen ersten dielektrischen Wellenleiter mit einem ersten Koppelabschnitt. Der erste Koppelabschnitt ist derjenige Abschnitt, der für die Kopplung beziehungsweise Datenübertragung primär genutzt wird.

Darüber hinaus besitzt die Datenübertragungsvorrichtung einen zweiten dielektrischen Wellenleiter, der gegenüber dem ersten Koppelabschnitt in einer Bewegungsrichtung bewegbar ist. Dies bedeutet, dass der zweite dielektrische Wellenleiter gegenüber dem ersten dielektrischen Wellenleiter bewegbar ist. Aufgrund der räumlichen Nähe zwischen dem ersten Koppelabschnitt und dem zweiten dielektrischen Wellenleiter ergibt sich eine Kopplung, die die kontaktlose Datenübertragung ermöglicht. Dabei verläuft der erste Koppelabschnitt vorzugsweise in Längsrichtung des zweiten dielektrischen Wellenleiters. Dies bedeutet aber auch, dass in diesem Fall der erste und zweite dielektrische Wellenleiter zueinander parallel verlaufen.

Der erste Koppelabschnitt hat einen ersten variablen Abstand gegenüber dem zweiten dielektrischen Wellenleiter. Dieser variable Abstand ergibt sich üblicherweise aus der Bewegung des zweiten dielektrischen Wellenleiters gegenüber dem ersten dielektrischen Wellenleiter. Aufgrund von mechanischen Toleranzen der beteiligten Bauteile ergibt sich typischerweise auch senkrecht zu der Bewegungsrichtung, in der sich die beiden Wellenleiter relativ zueinander bewegen, eine Abstandsvariation.

Die Datenübertragungsvorrichtung besitzt des Weiteren einen dritten dielektrischen Wellenleiter, der einen zweiten Koppelabschnitt und einen zweiten variablen Abstand zu dem zweiten dielektrischen Wellenleiter aufweist. Es besteht also auch eine direkte Kopplung zwischen dem dritten dielektrischen Wellenleiter und dem zweiten dielektrischen Wellenleiter.

Beispielsweise ist der dritte dielektrische Wellenleiter dem ersten dielektrischen in Bezug auf eine Senkrechte zur Bewegungsrichtung gegenüberliegend angeordnet.

Der erste dielektrische Wellenleiter ist fest in Bezug auf den dritten dielektrischen Wellenleiter angeordnet. Da sich der zweite dielektrische Wellenleiter relativ zu dem ersten dielektrischen Wellenleiter bewegt, resultiert daraus auch, dass sich der zweite dielektrische Wellenleiter gegenüber dem dritten dielektrischen Wellenleiter bewegt. Damit ist eine kontaktlose Datenübertragung von dem sich bewegenden zweiten dielektrischen Wellenleiter sowohl zu dem festen ersten dielektrischen Wellenleiter als auch zu dem festen dritten dielektrischen Wellenleiter möglich.

Der erste Koppelabschnitt und der zweite Koppelabschnitt sind in Bewegungsrichtung gleich lang und senkrecht zu der Bewegungsrichtung einander gegenüberliegend (vorzugsweise parallel) angeordnet. Die gleiche Länge der Koppelabschnitte hat den Vorteil, dass der erste und dritte dielektrische Wellenleiter im Prinzip gleichgeformt sein können, wodurch Kosteneinsparungen entstehen können. Darüber hinaus liegen sie senkrecht zu der Bewegungsrichtung einander gegenüber, wodurch unter Umständen Platzeinsparungen möglich sind, insbesondere, wenn die drei dielektrischen Wellenleiter im Koppelbereich, also über die Länge der Koppelabschnitte, zueinander parallel verlaufen. Falls die Koppelabschnitte gleich dimensioniert und zum zweiten dielektrischen Wellenleiter symmetrisch positioniert sind, ergibt sich für die beiden Einkopplungen jeweils ein gleicher Dynamikbereich, so dass die anschließende Signalverarbeitung auch gleich und damit kostengünstiger sein kann.

Gemäß einem Ausführungsbeispiel ist vorgesehen, dass in Bezug auf die Datenübertragung per optischer Kopplung entweder der zweite dielektrische Wellenleiter einer Sendeeinheit der Datenübertragungsvorrichtung und der erste gemeinsam mit dem dritten dielektrischen Wellenleiter einer gemeinsamen Empfangseinheit der Datenübertragungsvorrichtung zugeordnet sind, oder der zweite dielektrische Wellenleiter einer Empfangseinheit der Datenübertragungsvorrichtung und der erste und/oder dritte dielektrische Wellenleiter einer Sendeeinheit der Datenübertragungsvorrichtung zugeordnet sind. Diese beiden Varianten beziehen sich beispielsweise auf den Fall, dass von einer rotierenden Gantry nach außen Daten übertragen werden müssen oder umgekehrt nach innen zur rotierenden Gantry. Natürlich kann die jeweilige Sende- und Empfangsfunktion auch wechseln, wenn entsprechende Transceiver-Einheiten genutzt werden. Der Wechsel kann unter Umständen auch in sehr kurzen Zeitabständen erfolgen.

Für die Kopplung ist es generell erforderlich, dass die gekoppelten Wellenleiter eine bestimmte Nähe zueinander besitzen. Nur so ist es möglich, dass eine kritische Kopplungsstärke nicht unterschritten wird. Ein diesbezüglicher Maximalabstand hängt unter anderem auch von der Wellenlänge der zu übertragenden elektromagnetischen Signale ab.

Bei einem weiteren Ausführungsbeispiel sind der erste Koppelabschnitt und der zweite Koppelabschnitt senkrecht zu der Bewegungsrichtung symmetrisch zueinander angeordnet. Dies bedeutet, dass ihre Einkopplung gleich zueinander ist, wenn sie jeweils den gleichen Abstand zu dem bewegten zweiten dielektrischen Wellenleiter besitzen. Gegebenenfalls können auch alle Abschnitte des ersten dielektrischen Wellenleiters symmetrisch sein zu den entsprechenden Abschnitten des dritten dielektrischen Wellenleiters. In diesem Fall können die beiden Wellenleiter also gleichgeformt sein, was Fertigungsvorteile mit sich bringt.

In einer speziellen Ausgestaltung sind der erste Koppelabschnitt und der zweite Koppelabschnitt parallel zu dem zweiten dielektrischen Wellenleiter angeordnet. Dies bedeutet, dass über die Länge der Koppelabschnitte eine gleichmäßige Kopplung zwischen erstem und zweitem Wellenleiter, aber auch zwischen drittem und zweitem Wellenleiter gegeben sind.

In einem bevorzugten Ausführungsbeispiel ist vorgesehen, dass der zweite dielektrische Wellenleiter zwischen dem ersten und dritten dielektrischen Wellenleiter hindurchverläuft. Insbesondere verläuft der zweite dielektrische Wellenleiter zwischen dem ersten und zweiten Koppelabschnitt hindurch. Wenn also beispielsweise der erste und zweite Koppelabschnitt zusammen die Grenzen einer gemeinsamen Hülle bilden, verläuft der zweite dielektrische Wellenleiter durch das von der Hülle umgrenzte Volumen. Bei der gegenseitigen Abstandsvariation ergibt sich dann die Konstellation, dass der erste variable Abstand zwischen erstem und zweitem Wellenleiter zunimmt, wenn der zweite variable Abstand zwischen drittem und zweitem Wellenleiter abnimmt und umgekehrt.

Gemäß einem anderen Ausführungsbeispiel ist vorgesehen, dass der zweite dielektrische Wellenleiter auf einer Kreisbahn mit einem Kreiszentrum und einem Bahnradius ausgebildet ist, und die Koppelabschnitte in Umfangsrichtung gleich positioniert wie folgt angeordnet sind:
a) einer der beiden Koppelabschnitte radial außerhalb der Kreisbahn und der andere Koppelabschnitt radial innerhalb der Kreisbahn,
b) beide Koppelabschnitte mit dem Bahnradius vom Kreiszentrum entfernt axial beiderseits der Kreisbahn,
c) beide Koppelabschnitte radial innerhalb der Kreisbahn und zu dieser nach beiden Seiten axial versetzt, oder
d) beide Koppelabschnitte radial außerhalb der Kreisbahn und zu dieser nach beiden Seiten axial versetzt.

Der zweite dielektrische Wellenleiter befindet sich also auf einer Kreisbahn, die auf einer sich drehenden, runden Einheit, wie einer Gantry, realisiert sein kann. Beide Koppelabschnitte befinden sich auf der gleichen Position in Umfangsrichtung. Dies bedeutet, dass die Koppelabschnitte beide in ein und denselben Abschnitt des zweiten dielektrischen Wellenleiters ein- beziehungsweise auskoppeln. Dies bedeutet ferner im Falle des Auskoppelns aus dem zweiten dielektrischen Wellenleiter, dass die ausgekoppelten Signale im ersten und dritten Wellenleiter beziehungsweise im ersten und zweiten Koppelabschnitt die gleiche Phase besitzen. Bei der Variante a) stehen sich die beiden Koppelabschnitte radial gegenüber und der zweite dielektrische Wellenleiter läuft dazwischen durch. Damit kann beispielsweise einer radialen Unwucht eines Gantry begegnet werden.

Bei der Variante b) stehen sich die beiden Koppelabschnitte axial gegenüber und der zweite dielektrische Wellenleiter läuft dazwischen durch. In diesem Fall kann einer axialen Ungleichmäßigkeit bei der Signalauswertung entgegengewirkt werden.

Bei der Variante c) stehen sich die beiden Koppelabschnitte innerhalb der Kreisbahn axial gegenüber. Der zweite dielektrische Wellenleiter läuft dabei axial etwas versetzt radial über beide Koppelabschnitte hinweg. Mit dieser Kopplungskonstellation lassen sich auch axiale Unwuchten des zweiten dielektrischen Wellenleiters ausgleichen. Dabei ist jedoch zu beachten, dass sich der Abstand (bezogen auf die Wellenleiterzentren) vom zweiten Wellenleiter zum ersten Koppelabschnitt betragsmäßig anders ändert als der Abstand des zweiten Wellenleiters zum zweiten Koppelabschnitt.

Die Variante d) entspricht prinzipiell der Variante c), wobei die beiden Koppelabschnitte nur radial außerhalb der Kreisbahn angeordnet sind. Die Koppeleigenschaften sind also bei beiden Varianten im Wesentlichen gleich.

Gemäß einem weiteren Ausführungsbeispiel ist an den ersten und dritten Wellenleiter ein jeweiliger Empfänger zum Detektieren elektromagnetischer Signale im jeweiligen Wellenleiter angeordnet, und Signale der beiden Empfänger werden miteinander verknüpft. Dies bedeutet, dass zwar zwei Empfänger vorgesehen sind, aber deren Signale verknüpft, vorzugsweise addiert, werden. Je nach Abstand des zweiten dielektrischen Wellenleiters zu dem ersten beziehungsweise zweiten Koppelabschnitt ergeben sich unterschiedliche Kopplungsstärken und damit unterschiedliche Signale an beiden Empfängern. Es stehen somit immer zwei unterschiedlich starke Signale zur Verfügung, von denen das größere ausgenutzt werden kann.

Entsprechend einem weiteren Ausführungsbeispiel ist an dem ersten und dritten dielektrischen Wellenleiter ein einziger Empfänger angeordnet, der elektromagnetische Signale, die sowohl vom zweiten dielektrischen Wellenleiter in den ersten als auch in den dritten dielektrischen Wellenleiter eingekoppelt werden, empfängt. Es werden dabei also der erste und dritte Wellenleiter in Wellenrichtung nach der Kopplung mit dem zweiten Wellenleiter zusammengeführt und es wird ein Gesamtsignal mit einem einzigen Empfänger gewonnen beziehungsweise ausgewertet. Auf diese Weise lässt sich also ein Empfänger einsparen.

In einer konkreten Ausgestaltung ist vorgesehen, dass der erste und dritte dielektrische Wellenleiter in einem Abschnitt den zweiten dielektrischen Koppler bilden, mit dem ein elektromagnetisches Signal von dem dritten in den ersten dielektrischen Wellenleiter übertragbar ist, und der einzige Empfänger unmittelbar an dem ersten dielektrischen Wellenleiter angeordnet ist, um auch das von dem dritten in den ersten dielektrischen Wellenleiter eingekoppelte elektromagnetische Signal zu empfangen. Es ist also nur der erste dielektrische Wellenleiter mit einem Empfänger ausgestattet. Dieser empfängt das aus dem zweiten dielektrischen Wellenleiter in den ersten dielektrischen Wellenleiter eingekoppelte elektromagnetische Signal direkt. Das in den dritten dielektrischen Wellenleiter eingekoppelte elektromagnetische Signal wird zunächst zu dem zweiten Koppler geführt und dort in den ersten dielektrischen Wellenleiter eingekoppelt, bevor es von dem einzigen Empfänger detektiert werden kann. Es ist also kein Empfänger am dritten dielektrischen Wellenleiter notwendig.

In einer besonders bevorzugten Ausgestaltung ist der Koppelabschnitt des ersten dielektrischen Wellenleiters von dem zweiten Koppler ein erstes Maß beabstandet, der Koppelabschnitt des dritten dielektrischen Wellenleiters ist von dem zweiten Koppler ein zweites Maß beabstandet, und das zweite Maß entspricht einer Summe des ersten Maßes und eines Vielfachen einer für die Datenübertragung vorgegebenen Wellenlänge in den dielektrischen Wellenleitern. Dies hat zur Folge, dass die beiden elektromagnetischen Signale bei Ankunft an dem zweiten Koppler synchronisiert sind, denn der dritte dielektrische Wellenleiter ist um ein Vielfaches (einschließlich 0 und 1) der für die Datenübertragung vorgesehenen Wellenlänge länger als der erste Wellenleiter betrachtet vom Ende des (ersten) Kopplers zum Beginn oder zur Mitte des zweiten Kopplers.

In einer alternativen Ausführungsform ist vorgesehen, dass der erste und dritte dielektrische Wellenleiter zu einem dielektrischen Kombinierer geführt sind oder in einem Abschnitt den dielektrischen Kombinierer bilden, mit dem ein elektromagnetisches Signal sowohl von dem ersten als auch von dem dritten dielektrischen Wellenleiter in den einzigen Empfänger leitbar ist. Der erste und der dritte dielektrische Wellenleiter werden also zu einem einzigen Wellenleiter kombiniert beziehungsweise zusammengeführt und der einzige Empfänger registriert dann elektromagnetische Signale in dem kombinierten Wellenleiterabschnitt. Im Falle der Umkehrung der Datenübertragung kann der Kombinierer auch als Teiler gesehen werden.

Erfindungsgemäß wird auch eine Bildgebungsvorrichtung bereitgestellt, die eine Datenübertragungseinheit aufweist, wie sie oben beschrieben wurde. Damit kann eine gegenüber dem Stand der Technik alternative oder vereinfachte Bildgebungsvorrichtung aufgebaut werden.

In einem spezifischen Ausführungsbeispiel ist vorgesehen, dass der zweite dielektrische Wellenleiter Teil einer rotierbaren Gantry mit einer Gantry-Tragstruktur und der erste sowie der dritte dielektrische Wellenleiter Teil einer feststehenden Basis (im vorliegenden Dokument auchals Halterahmen bezeichnet) ist. Dabei kann der zweite dielektrische Wellenleiter als Teil der Sendeeinheit an der Gantry-Tragstruktur fixiert sein. Somit lassen sich mit hoher Bandbreite zuverlässig Daten von der Gantry zu der feststehenden Basis übertragen und umgekehrt.

Die Bildgebungsvorrichtung kann als Computertomograph oder als Magnetresonanztomograph ausgebildet sein. Beide Tomographen besitzen entsprechende Gantrys und somit den Bedarf, Daten von dem Gantry mit hoher Geschwindigkeit nach außen zu übertragen.

Die obige Aufgabe wird erfindungsgemäß auch gelöst durch ein Verfahren zur kontaktlosen Datenübertragung (optisch), aufweisend
- Bewegen eines zweiten dielektrischen Wellenleiters bei der Datenübertragung gegenüber einem ersten Koppelabschnitt eines ersten dielektrischen Wellenleiters in einer Bewegungsrichtung, (z.B. in Längsrichtung des zweiten dielektrischen Wellenleiters) wobei ein erster Abstand des zweiten dielektrischen Wellenleiters (z.B. Seitenfläche zu gegenüberliegender Seitenfläche oder Mittellinie zu Mittellinie; von null verschieden) zu dem ersten Koppelabschnitt variiert, und wobei ein zweiter Abstand eines zweiten Koppelabschnitts eines dritten dielektrischen Wellenleiters (dem ersten dielektrischen Wellenleiter gegenüberliegt und) zu dem zweiten dielektrischen Wellenleiter variiert, wobei
- der erste dielektrische Wellenleiter fest in Bezug auf den dritten dielektrischen Wellenleiter angeordnet ist,
   wobei
- der erste Koppelabschnitt und der zweite Koppelabschnitt in der Bewegungsrichtung gleich lang sind und senkrecht zu der Bewegungsrichtung einander gegenüberliegen.

In Bezug auf das Verfahren ergeben sich sinngemäß die gleichen Vorteile und Weiterbildungsmöglichkeiten wie für die obige Bildgebungsvorrichtung beziehungsweise die obige Datenübertragungseinheit.

Es wird also ein elektromagnetisches Signal über eine optische Richtkopplung zwischen dem zweiten dielektrischen Wellenleiter einerseits und dem ersten bzw. dritten dielektrischen Wellenleiter andererseits übertragen. Unter der optischen Richtkopplung ist hierbei eine Kopplung zu verstehen, in welcher ein durch einen Wellenleiter geführtes optisches Signal, dessen Moden teilweise außerhalb des Wellenleiters propagieren, in einen zum Wellenleiter wenigstens streckenweise parallelen weiteren Wellenleiter "übersprechen" und hierdurch auch dort für ein propagierendes optisches Signal sorgen.

Die Datenübertragungsvorrichtung kommt bevorzugt in einer Bildgebungsvorrichtung zum Einsatz, wie sie insbesondere in der DE 10 2016 208 539 A1, oder in der DE 10 2015 223 068 A1 beschrieben ist. Dabei ersetzt dann die hier beschriebene Datenübertragungsvorrichtung die jeweils in diesen Druckschriften beschriebene Datenübertragungseinheiten oder Übertragungsstrecken. Es wird daher an dieser Stelle ausdrücklich auf die Beschreibungen in beiden Druckschriften Bezug genommen.

Ausführungsbeispiele der Erfindung werden nachfolgend anhand schematischer Zeichnungen näher erläutert. Darin zeigen:
- FIG 1: eine schematische Ansicht eines Computertomographen;
- FIG 2: ein Ausführungsbeispiel eines Kopplers;
- FIG 3: ein weiteres Ausführungsbeispiel eines Kopplers;
- FIG 4: ein noch anderes Ausführungsbeispiel eines Kopplers;
- FIG 5: eine Schnittansicht der Koppler der FIG 2 bis 4 in einer Mittelstellung;
- FIG 6: die Ansicht von FIG 5 bei axialem Versatz des zweiten Wellenleiters;
- FIG 7: die Ansicht von FIG 5 bei radialem Versatz des zweiten Wellenleiters; und
- FIG 8: eine alternative Anordnung der Wellenleiter des Kopplers.

Die nachfolgend näher geschilderten Ausführungsbeispiele stellen bevorzugte Ausführungsformen der vorliegenden Erfindung dar.

Einander entsprechende Teile sind in allen Figuren jeweils mit den gleichen Bezugszeichen versehen.

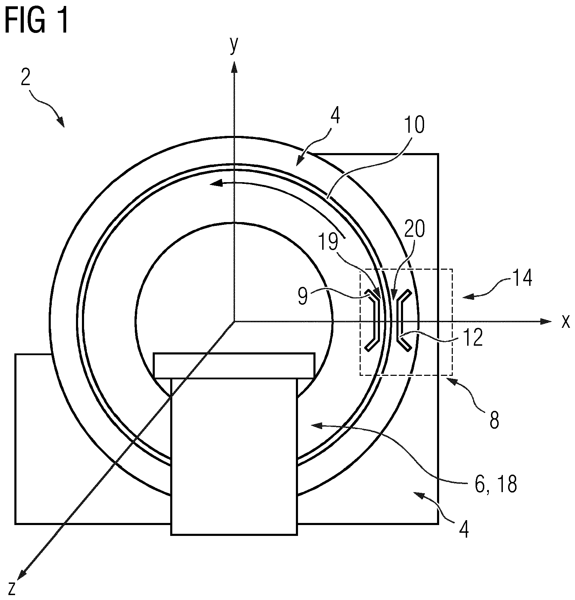

Eine nachfolgend exemplarisch beschriebene und in FIG 1 skizzierte Bildgebungsvorrichtung ist als Computertomograph 2 ausgebildet und weist eine feststehende Basis 4 sowie eine rotierbare Gantry 6 auf. Hierbei ist die Gantry 6 nach an sich bekanntem Prinzip zur Generierung von Bilddaten mittels eines nicht mit abgebildeten Röntgendetektors ausgebildet und dementsprechend werden dann im Betrieb des Computertomographen 2 Daten generiert, die während der Rotation der Gantry 6 von der rotierbaren Gantry 6 zur feststehenden Basis 4 zu übertragen sind und mittels einer Datenübertragungseinheit 8 übertragen werden.

Teil dieser Datenübertragungseinheit 8 sind ein erster dielektrischer Wellenleiter 9, ein zweiter dielektrischer Wellenleiter 10 sowie ein dritter dielektrischer Wellenleiter 12. Der erste und dritte dielektrischer Wellenleiter 9 und 12 sind an der Basis 4 befestigt, während der zweite dielektrische Wellenleiter 10 an der Gantry 6 befestigt ist. Die dielektrischen Wellenleiter 9, 10 und 12 sind derart zueinander angeordnet, dass zwischen dem ersten dielektrischen Wellenleiter 9 und dem zweiten dielektrischen Wellenleiter 10 ein Luftspalt 19 sowie zwischen dem zweiten dielektrischen Wellenleiter 10 und dem dritten dielektrischen Wellenleiter 12 ein Luftspalt 20 bestehen. Der jeweilige Luftspalt 19 bzw. 20 kann im Mittel etwa 1 mm betragen, und bei Rotation der Gantry 6 in einem Bereich zwischen 0,5 mm und 1,5 mm variieren.

Über diesen Luftspalt 20 hinweg lassen sich nach an sich bekanntem Prinzip Bilddaten vom zweiten dielektrischen Wellenleiter 10 zum ersten und dritten dielektrischen Wellenleiter 9 und 12 übertragen und somit von der rotierbaren Gantry 6 zur feststehenden Basis 4. Der zweite dielektrische Wellenleiter 10 ist somit Teil einer Sendeeinheit, während der erste und dritte dielektrische Wellenleiter 9, 12 Teil einer Empfangseinheit sind. Somit bilden der zweite dielektrische Wellenleiter 10 und der erste sowie dritte dielektrische Wellenleiter 9, 12 einen Koppler 14 mit aus, der die Basis 4 und die Gantry 6 signaltechnisch koppelt.

In bevorzugter Ausgestaltung ist der zweite dielektrische Wellenleiter 10 dabei ringförmig gestaltet oder nimmt im montierten Zustand eine ringförmige Gestalt an, wie dies in FIG 1 angedeutet ist. Der zweite dielektrische Wellenleiter 10 verläuft somit im montierten Zustand um den Umfang der rotierbaren Gantry 6 herum und ist dabei bevorzugt in einer Nut versenkt angeordnet. Jene Nut 16 ist hierbei Teil einer Gantry-Tragstruktur 18 und bildet bevorzugt einen zweiten Teil einer Steckverbindung aus, mittels derer der zweite dielektrische Wellenleiter 10 an der Gantry-Tragstruktur 18 befestigt ist.

Der erste dielektrische Wellenleiter 9 und der dritte dielektrische Wellenleiter 12 besitzen im vorliegenden Beispiel in der axialen Ansicht in etwa eine C-förmige Gestalt. Beide Wellenleiter 9 und 12 sind hier spiegelsymmetrisch zum zweiten dielektrischen Wellenleiter 10 angeordnet. Der erste dielektrische Wellenleiter 9 befindet sich radial innerhalb des zweiten dielektrischen Wellenleiters 10 und der dritte dielektrische Wellenleiter 12 radial außerhalb. Die offene Seite der C-Form des ersten dielektrischen Wellenleiters 9 zeigt zum Zentrum der Gantry 6, während die offene Seite des dritten dielektrischen Wellenleiters 12 radial nach außen gerichtet ist.

In einer alternativen Ausführungsform befinden sich erster bis dritter dielektrischer Wellenleiter 9, 10, 12 auf der gleichen radialen Position. In axialer Richtung befindet sich dabei der zweite dielektrische Wellenleiter 10 zwischen dem ersten und dritten dielektrischen Wellenleiter 9, 12. Die Luftspalte beziehungsweise Abstände zwischen den Wellenleitern könnten in der gleichen Größenordnung liegen wie beim vorhergehenden Ausführungsbeispiel.

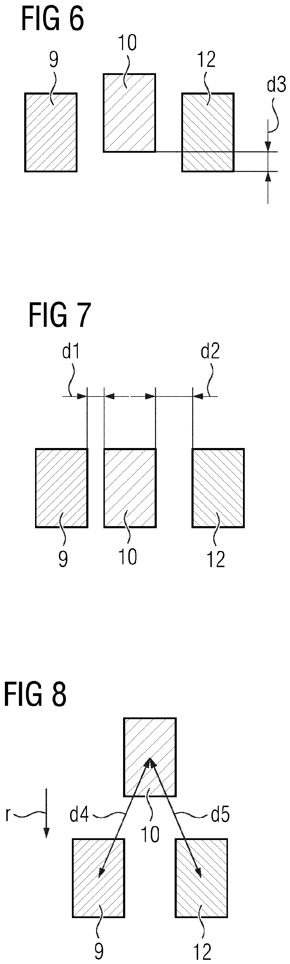

In einer Mischform (vgl. FIG 8) liegen der erste und dritte dielektrische Wellenleiter 9, 12 auf gleicher radialer Position und der zweite dielektrische Wellenleiter 10 liegt radial darunter oder darüber. Hier könnte sich der Abstand zweier Wellenleiter auf die Distanz der Zentren zweier Wellenleiter beziehen. Die Kopplungsstärke könnte dann als Funktion dieser Distanzen betrachtet werden.

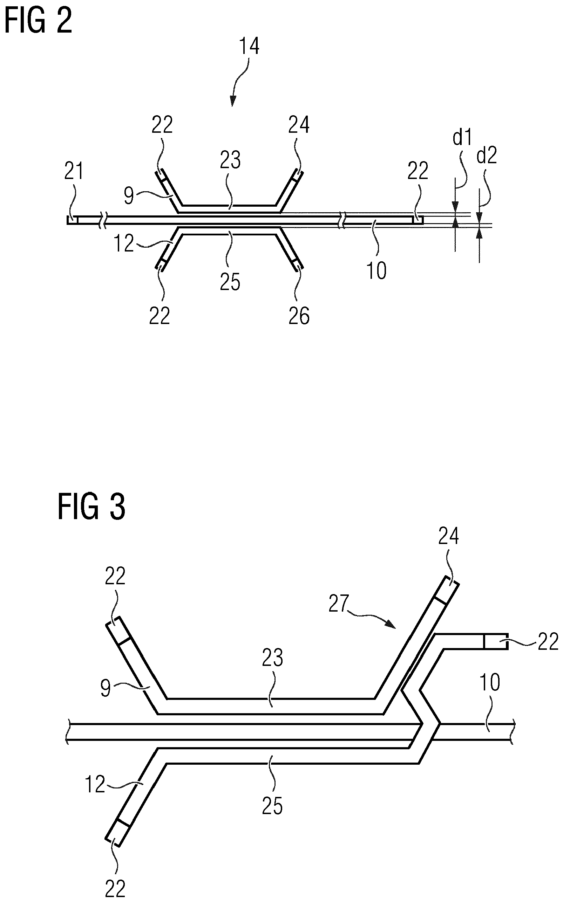

In FIG 2 ist der Koppler 14 entsprechend dem Ausführungsbeispiel von FIG 1 vergrößert dargestellt. Der zweite dielektrische Wellenleiter 10 ist hier ohne Gantry 6 schemenhaft als gerade Linie dargestellt. Dies entspricht jedoch nur einer vereinfachten Darstellung, insbesondere dann, wenn der zweite dielektrische Wellenleiter 10 ringförmig ausgebildet ist und die Mittelachse senkrecht zur Bildebene angeordnet ist. Alternativ kann sich die Mittelachse (Drehachse der Gantry 6) auch parallel zur Bildebene erstrecken, so dass der ringförmige zweite dielektrische Wellenleiter 10 in der Draufsicht tatsächlich eine gerade Linie darstellt. An einem Ende des zweiten dielektrischen Wellenleiters 10 ist ein Sender 21 vorgesehen, der auch Empfangsfunktionalität besitzen kann. Am anderen Ende des zweiten dielektrischen Wellenleiters 10 befindet sich beispielsweise ein Abschluss 22.

Auf beiden Seiten des zweiten dielektrischen Wellenleiters 10 sind gegenüberliegend der erste Wellenleiter 9 und der dritte Wellenleiter 12 angeordnet. Der erste dielektrische Wellenleiter 9 besitzt beispielsweise in seinem Mittelbereich einen ersten Koppelabschnitt 23. In seinen Endabschnitten 24 kann er von dem zweiten dielektrischen Wellenleiter 10 weggeführt sein, so dass sich die in FIG 2 schematisch dargestellte C-Form des ersten dielektrischen Wellenleiters 9 ergibt. An seinem einen Ende kann ein Abschluss 22 und an seinem anderen Ende ein erster Empfänger 24 angeordnet sein. Der Sender 1 strahlt Licht durch den zweiten dielektrischen Wellenleiter 10, das in den ersten dielektrischen Wellenleiter 9 einkoppelt und darin zum ersten Empfänger 24 geführt wird. Der erste Empfänger 24 befindet sich daher in Strahlrichtung nach dem ersten Koppelabschnitt 23.

Der dritte Wellenleiter 12 ist achsensymmetrisch zum ersten dielektrischen Wellenleiter 9 geformt und angeordnet. Die Spiegelachse symbolisiert der zweite dielektrische Wellenleiter 10. Dem entsprechend besitzt der dritte dielektrische Wellenleiter 12 einen zweiten Koppelabschnitt 25 in seinem Mittelbereich und an seinem einen Ende einen Abschluss 22 sowie am anderen Ende einen zweiten Empfänger 26.

Der erste Koppelabschnitt 23 und der zweite Koppelabschnitt 25 sind vorzugsweise parallel zueinander angeordnet und zwischen Ihnen verläuft der zweite dielektrische Wellenleiter 10. Der erste Koppelabschnitt 23 und der zweite dielektrische Wellenleiter 10 besitzen zueinander eine Distanz d1. Diese Distanzen d1 und d2 variieren mit der Exzentrizität beziehungsweise Unwucht der Gantry 6 beziehungsweise des darauf montierten zweiten dielektrischen Wellenleiters 10. Diese Unwucht ergibt sich, wenn die Rotationsachse der Gantry 6 nicht einer seiner Hauptträgheitsachsen entspricht. Die Summe der Distanzen d1 + d2 ist jedoch konstant.

Die Koppelstärke zwischen dem zweiten dielektrischen Wellenleiter 10 und dem ersten dielektrischen Wellenleiter 9 beziehungsweise dem dritten dielektrischen Wellenleiter 12 hängt von der jeweiligen Distanz d1 beziehungsweise d2 ab. Die Stärke der Kopplung zwischen den jeweiligen dielektrischen Wellenleiterpaaren entspricht einer komplexen, nicht monotonen Funktion in Abhängigkeit von der jeweiligen Distanz d1 beziehungsweise d2. Die für die jeweilige Kopplung relevanten physikalischen Interaktionsprozesse besitzen eine funktionale Abstandsabhängigkeit, welche nicht notwendigerweise zu einem monotonen Anstieg der Kopplungsstärke bei abnehmendem Abstand führt, sondern zu der genannten komplexen funktionellen Abhängigkeit beispielsweise infolge von destruktiven Interferenzen von Wellenanteilen des Signals.

Zur Reduktion dieser Abhängigkeit wird das Licht des rotierenden zweiten dielektrischen Wellenleiters 10 zu beiden Seiten in den jeweiligen gering beabstandeten Koppelabschnitt 23, 25 beziehungsweise dielektrischen Wellenleiter 9, 12 eingekoppelt. Die Signale der beiden Empfänger 24 und 26 können beispielsweise addiert werden, wodurch die Abstandsabhängigkeit der Kopplungsstärke vermindert wird.

Der erste dielektrische Wellenleiter 9 und der dritte dielektrische Wellenleiter 12 beziehungsweise deren Koppelabschnitte 23 und 25 sind fest zueinander angeordnet. Dies bedeutet, dass die Summe der Abstände d1 und d2 konstant ist, da die Breite des zweiten dielektrischen Wellenleiters 10 über seiner Länge als gleichförmig angesehen werden kann. Wenn also bei einer Unwucht der Gantry 6 zu einem Zeitpunkt der Abstand d1 sinkt, steigt der Abstand d2 und umgekehrt. Dies bedeutet jedoch nicht, dass die Summe der Kopplungsstärken konstant ist. Vielmehr bestimmen, wie oben dargelegt, einschlägige Nichtlinearitäten die (gesamte) Kopplungsstärke.

FIG 3 zeigt eine alternative Ausführungsform, bei der gegenüber der vorhergehenden Ausführungsform auf den zweiten Empfänger 26 verzichtet werden kann. Der erste Wellenleiter 9 besitzt im Wesentlichen die gleiche Form wie im Ausführungsbeispiel von FIG 2. An einem Ende besitzt er den Abschluss 22 und am anderen Ende den ersten Empfänger 24. Der zweite Koppelabschnitt 25 des dritten Wellenleiters 12 verläuft hier ebenfalls parallel zu dem ersten Koppelabschnitt 23 des ersten dielektrischen Wellenleiters 9. Auch der zweite dielektrische Wellenleiter 10 bewegt sich durch die beiden parallelen Koppelabschnitte 23 und 25.

Anders als in dem Beispiel von FIG 2 besitzen der erste und der dritte dielektrische Wellenleiter 9 und 12 zusätzlich einen gemeinsamen dielektrischen Koppelabschnitt 27. Dort verlaufen der erste und der dritte dielektrische Wellenleiter 9 und 12 ein Stück parallel, ohne dass der zweite dielektrische Wellenleiter 10 dazwischen hindurchläuft. In dem gemeinsamen dielektrischen Koppelabschnitt 27 erfolgt eine direkte optische Kopplung zwischen beiden Wellenleitern 9 und 12. Dies bedeutet, dass das Licht, das in den zweiten dielektrischen Wellenleiter 10 eingespeist und im Koppelabschnitt 25 in den dritten dielektrischen Wellenleiter eingekoppelt wird, in dem gemeinsamen dielektrischen Koppelabschnitt 27 in den ersten dielektrischen Wellenleiter 9 gekoppelt wird. Gleichzeitig wird Licht beziehungsweise elektromagnetische Strahlung von dem zweiten dielektrischen Wellenleiter 10 nach wie vor direkt in dem ersten Koppelabschnitt 23 in den ersten dielektrischen Wellenleiter 9 eingekoppelt. Der erste Empfänger 24 empfängt somit das Licht beziehungsweise die elektromagnetische Strahlung, die in den ersten dielektrischen Wellenleiter 9, aber auch das Licht beziehungsweise die elektromagnetische Strahlung, die in den dritten dielektrischen Wellenleiter 12 eingekoppelt wird. Der dritte dielektrische Wellenleiter 12 benötigt an seinen Enden somit allenfalls jeweils einen Abschluss 22.

Da sich die beiden Koppelabschnitte 23 und 25 auf gegenüberliegenden Seiten des zweiten dielektrischen Wellenleiters 10 befinden, muss der dritte dielektrische Wellenleiter 12 über den zweiten dielektrischen Wellenleiter 10 auf die Seite des ersten dielektrischen Wellenleiters 9 geführt werden, um dort den gemeinsamen dielektrischen Koppelabschnitt 27 realisieren zu können.

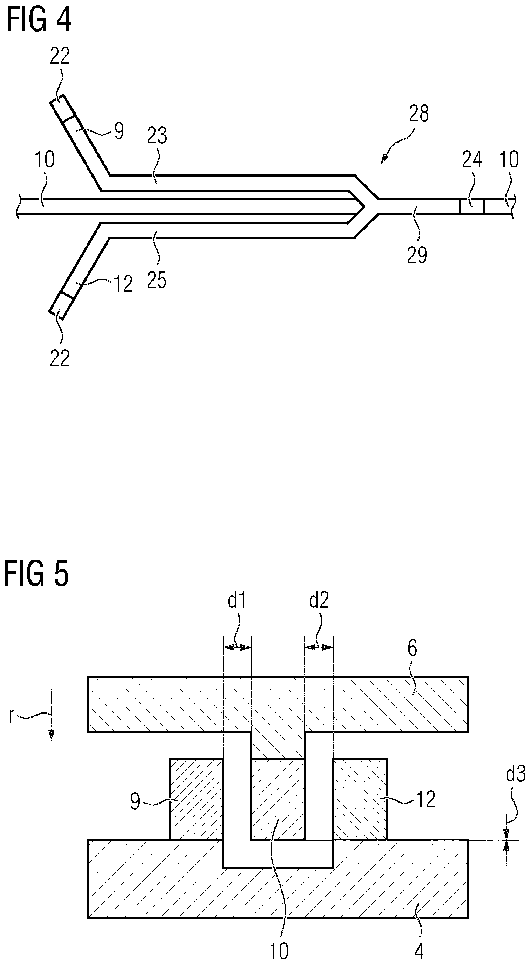

Bei der Ausführungsform gemäß FIG 4 ist ebenfalls für beide dielektrischen Wellenleiter 9 und 12 nur ein einziger Empfänger 24 notwendig. Der Aufbau der Kopplungsanordnung entspricht im Wesentlichen demjenigen von FIG 3. Auch hier verläuft der zweite dielektrische Wellenleiter 10 zwischen den parallelen Koppelabschnitten 23 und 25 des ersten und dritten dielektrischen Wellenleiters 9 und 12. Anstelle des gemeinsamen dielektrischen Koppelabschnitts 27 ist hier jedoch ein dielektrischer Kombinierer 28 vorgesehen, der den ersten dielektrischen Wellenleiter 9 und den dritten dielektrischen Wellenleiter 12 zu einem gemeinsamen Wellenleiter 29 kombiniert. Am Ende dieses gemeinsamen Wellenleiters 29 ist der erste Empfänger 24 angeordnet.

Der Kombinierer 28 führt den ersten und dritten dielektrischen Wellenleiter 9 und 12 oberhalb des zweiten dielektrischen Wellenleiters 10 (z. B. radial außerhalb von dessen Kreisbahn) zusammen. Damit werden sowohl die in den ersten Koppelabschnitt 23 als auch in den zweiten Koppelabschnitt 25 eingekoppelten Lichtanteile in dem gemeinsamen Wellenleiter 29 zusammengeführt. Der Kombinierer 28 kann beispielsweise durch 3D-Druck realisiert werden, so dass sich die Wellenleiter in unterschiedlichen Ebenen befinden.

Die Längen und Abstände der einzelnen Wellenleiter in dem gemeinsamen dielektrischen Koppelabschnitt 27 sind so zu wählen, dass beim Empfang der Maximalwert der Dämpfung möglichst gering ist. Insbesondere sollten die Längen der Koppelabschnitte ein Mehrfaches der gewählten Wellenlänge in dem Wellenleitermaterial betragen (beispielsweise beträgt die Wellenlänge bei 60 GHz in dem Wellenleitermaterial 3 mm).

Die Diagramme der FIG 5, 6 und 7 zeigen einen Querschnitt beispielsweise durch eine der Wellenleiteranordnungen gemäß den FIG 2 bis 4. Der Schnitt verläuft senkrecht zu den Bildebenen dieser FIG 2 bis 4. Bei kreisrunder Gantry verläuft der Schnitt in radialer Richtung.

FIG 5 zeigt einen Teil der Gantry 6 und einen Teil der Basis (beziehungsweise Halterahmen) 4 beispielsweise eines Computertomographen. Bezogen auf die kreisförmige Gantry 6 verläuft die radiale Koordinate r in FIG 5 nach unten. An der Gantry 6 ist der zweite dielektrische Wellenleiter 10 befestigt. Der erste dielektrische Wellenleiter 9 befindet sich in FIG 5 links von dem zweiten dielektrischen Wellenleiter 10 und der dritte Wellenleiter 12 befindet sich rechts von dem zweiten dielektrischen Wellenleiter 10. Die drei Wellenleiter 9, 10 und 12 befinden sich hier in Bezug auf die radiale Koordinate r auf gleicher Höhe.

FIG 5 zeigt eine Soll-Zustand der Wellenleiteranordnung der drei Wellenleiter 9, 10 und 12. Der Abstand d1 zwischen erstem und zweitem dielektrischen Wellenleiter 9, 10 entspricht dem Abstand d2 zwischen dem zweiten und dritten dielektrischen Wellenleiter 10 und 12. Da sich die Wellenleiter alle auf gleicher radialer Höhe befinden, ist der radiale Abstand d3 zweier benachbarter Wellenleiter jeweils gleich 0.

In den FIG 6 und 7 ist auf die Darstellung der Gantry 6 und der Basis 4 verzichtet. Es sind lediglich die drei Wellenleiter 9, 10 und 12 im Schnitt dargestellt.

FIG 6 zeigt schematisch eine radiale Exzentrizität der Gantry 6 beziehungsweise des zweiten dielektrischen Wellenleiters 10. Der zweite dielektrische Wellenleiter 10 axial zwischen den beiden Wellenleitern 9 und 12 besitzt zu diesen zumindest in einer Drehposition einen gewissen radialen Abstand d3 größer oder kleiner als 0. Gegenüber der idealen Kopplungsanordnung von FIG 5 ist aufgrund dieser radialen Verschiebung des zweiten dielektrischen Wellenleiters 10 die Kopplungsstärke reduziert.

In FIG 7 ist der Fall einer axialen Unwucht der Gantry 6 schematisch angedeutet. Der zweite dielektrische Wellenleiter 10 befindet sich nicht mehr mittig zwischen dem ersten und dritten dielektrischen Wellenleiter 9 und 12. Vielmehr ist hier der Abstand d1 kleiner als der Abstand d2. In diesem Fall kann die Kopplung zwischen zweitem und erstem dielektrischen Wellenleiter 10, 9 größer sein als die Kopplungsstärke zwischen zweitem und drittem dielektrischen Wellenleiter 9, 12 (Nichtlinearitäten außeracht gelassen).

Die FIG 5 bis 7 verdeutlichen die Funktionsweise der Wellenleiterkopplung. Bewegt sich der zweite dielektrische Wellenleiter 10 nach links oder rechts, so ändern sich die Abstände d1 und d2 und somit auch die Dämpfung im Wesentlichen genau gegenläufig. Es kommt somit nie zu einer sehr hohen Dämpfung/Nullstelle in der Koppeldämpfung, die einen Abbruch der Signalübertragung zur Folge hätte.

Die Abstände und Längen der Koppler können so dimensioniert werden, dass beim Empfang der Maximalwert der Dämpfung möglichst gering ist.

Ein Versatz der Gantry beziehungsweise des zweiten dielektrischen Wellenleiters 10 in radialer Richtung (d3) ist in der Regel weniger von Bedeutung. Zum einen sind die Anordnungen meist so ausgelegt, dass in radialer Richtung weniger mechanische Toleranzen bei der Gantry-Rotation auftreten. Zum anderen führt dieser radiale Versatz der dielektrischen Wellenleiter auch nur zu einer verhältnismäßig geringen Kopplungsvariation.

FIG 8 zeigt ein weiteres Ausführungsbeispiel einer Wellenleiteranordnung in einem Schnitt entsprechend den FIG 5 bis 7. Die Zentren der drei Wellenleiter sind hier dreiecksförmig angeordnet. Die Zentren zwischen erstem und zweitem dielektrischen Wellenleiter 9, 10 besitzen den Absatz d4 und die Zentren des zweiten und dritten dielektrischen Wellenleiters 10, 12 besitzen den Abstand d5. Die Summe der Abstände d4 + d5 ist nicht konstant, wenn sich der zweite dielektrische Wellenleiter 10 gegenüber dem ersten und dritten dielektrischen Wellenleiter 9, 12 bewegt. Dennoch kann auch mit dieser Anordnung ein höherer Kopplungsgrad in den meisten Bewegungsphasen erreicht werden als bei nur zwei sich gegenüberstehenden Kopplungsleitern. Alternativ zu der Anordnung von FIG 8 kann der zweite dielektrische Wellenleiter 10 auch radial außerhalb der anderen beiden Wellenleiter 9, 12 angeordnet sein.

In vorteilhafter Weise kann durch die oben in den Ausführungsbeispielen gezeigten Kopplungsanordnungen eine robustere Kopplung erzielt werden, auch wenn die Bewegung eines Wellenleiters nicht gleichmäßig ist.

## Patentansprüche

1. Datenübertragungsvorrichtung (8) zur kontaktlosen Datenübertragung, aufweisend
- einen ersten dielektrischen Wellenleiter (9) mit einem ersten Koppelabschnitt (23),
- einen zweiten dielektrischen Wellenleiter (10), der gegenüber dem ersten Koppelabschnitt (23) in einer Bewegungsrichtung bewegbar ist und einen ersten variablen Abstand (d1) zu dem ersten Koppelabschnitt (23) aufweist, und
- einen dritten dielektrischen Wellenleiter (12), der einen zweiten Koppelabschnitt (25) und einen zweiten variablen Abstand (d2) zu dem zweiten dielektrischen Wellenleiter (10) aufweist, wobei
- der erste dielektrische Wellenleiter (9) fest in Bezug auf den dritten dielektrischen Wellenleiter (12) angeordnet ist,
**dadurch gekennzeichnet, dass**
- der erste Koppelabschnitt (23) und der zweite Koppelabschnitt (25) in der Bewegungsrichtung gleich lang sind und senkrecht zu der Bewegungsrichtung einander gegenüber liegen.

2. Datenübertragungsvorrichtung nach Anspruch 1, wobei in Bezug auf die Datenübertragung per optischer Kopplung entweder
- der zweite dielektrische Wellenleiter (10) einer Sendeeinheit der Datenübertragungsvorrichtung (8) und der erste gemeinsam mit dem dritten dielektrischen Wellenleiter (9, 12) einer gemeinsamen Empfangseinheit der Datenübertragungsvorrichtung (8) zugeordnet sind, oder
- der zweite dielektrische Wellenleiter (10) einer Empfangseinheit der Datenübertragungsvorrichtung (8) und der erste und/oder dritte dielektrische Wellenleiter (9, 12) einer Sendeeinheit der Datenübertragungsvorrichtung (8) zugeordnet sind.

3. Datenübertragungsvorrichtung nach Anspruch 1 oder 2, wobei der erste Koppelabschnitt (23) und der zweite Koppelabschnitt (25) senkrecht zu der Bewegungsrichtung symmetrisch zueinander angeordnet sind.

4. Datenübertragungsvorrichtung nach Anspruch 3, wobei der erste und zweite Koppelabschnitt (23, 25) parallel zu dem zweiten dielektrischen Wellenleiter (10) angeordnet sind.

5. Datenübertragungsvorrichtung nach einem der vorhergehenden Ansprüche, wobei der zweite dielektrische Wellenleiter (10) zwischen dem ersten und dritten dielektrischen Wellenleiter (9, 12) hindurch verläuft.

6. Datenübertragungsvorrichtung nach einem der Ansprüche 3 bis 5, wobei der zweite dielektrische Wellenleiter (10) auf einer Kreisbahn mit einem Kreiszentrum und einem Bahnradius ausgebildet ist, und die Koppelabschnitte (23, 25) in Umfangsrichtung gleich positioniert wie folgt angeordnet sind:
a) einer der beiden Koppelabschnitte radial außerhalb der Kreisbahn und der andere Koppelabschnitt radial innerhalb der Kreisbahn,
b) beide Koppelabschnitte mit dem Bahnradius vom Kreiszentrum entfernt axial beiderseits der Kreisbahn,
c) beide Koppelabschnitte radial innerhalb der Kreisbahn und zu dieser nach beiden Seiten axial versetzt, oder
d) beide Koppelabschnitte radial außerhalb der Kreisbahn und zu dieser nach beiden Seiten axial versetzt.

7. Datenübertragungsvorrichtung nach einem der vorhergehenden Ansprüche, wobei an dem ersten und dritten Wellenleiter (9, 12) ein jeweiliger Empfänger (24, 26) zum Detektieren elektromagnetischer Signale im jeweiligen Wellenleiter angeordnet ist, und Signale der beiden Empfänger (24, 26) miteinander verknüpft werden.

8. Datenübertragungsvorrichtung nach einem der Ansprüche 1 bis 6, wobei an dem ersten und dritten dielektrischen Wellenleiter (9, 12) ein einziger Empfänger (24) angeordnet ist, der elektromagnetische Signale, die sowohl vom zweiten dielektrischen Wellenleiter (10) in den ersten als auch in den dritten dielektrischen Wellenleiter (9, 12) eingekoppelt werden, empfängt.

9. Datenübertragungsvorrichtung nach Anspruch 8, wobei der erste und dritte dielektrische Wellenleiter (9, 12) in einem Abschnitt einen zweiten dielektrischen Koppler (27) bilden, mit dem ein elektromagnetisches Signal von dem dritten in den ersten dielektrische Wellenleiter übertragbar ist, und der einzige Empfänger (24) unmittelbar an dem ersten dielektrischen Wellenleiter (9) angeordnet ist, um das von dem dritten in den ersten dielektrischen Wellenleiter eingekoppelte elektromagnetische Signal zu empfangen.

10. Datenübertragungsvorrichtung nach Anspruch 9, wobei der Koppelabschnitt (23) des ersten dielektrischen Wellenleiters (9) von dem zweiten Koppler (27) ein erstes Maß beabstandet ist, der Koppelabschnitt (25) des dritten dielektrischen Wellenleiters (12) von dem zweiten Koppler (27) ein zweites Maß beabstandet ist, und das zweite Maß einer Summe des ersten Maßes und eines Vielfachen einer für die Datenübertragung vorgegebenen Wellenlänge in den dielektrischen Wellenleitern entspricht.

11. Datenübertragungsvorrichtung nach Anspruch 8, wobei der erste und dritte dielektrische Wellenleiter (9, 12) zu einem dielektrischen Kombinierer (28) geführt sind oder in einem Abschnitt den dielektrischen Kombinierer bilden, mit dem ein elektromagnetisches Signal sowohl von dem ersten als auch von dem dritten dielektrischen Wellenleiter (9, 12) in den einzigen Empfänger (24) leitbar ist.

12. Bildgebungsvorrichtung (2) aufweisend eine Datenübertragungseinheit (8) nach einem der vorherigen Ansprüche.

13. Bildgebungsvorrichtung (2) nach Anspruch 12, wobei der zweite dielektrischen Wellenleiter (10) Teil einer rotierbaren Gantry (6) mit einer Gantry-Tragstruktur (18) und der erste sowie der dritte dielektrische Wellenleiter (9, 12) Teil einer feststehenden Basis (4) ist.

14. Bildgebungsvorrichtung nach Anspruch 12 oder 13, die als Computertomograph oder als Magnetresonanztomograph ausgebildet ist.

15. Verfahren zur kontaktlosen Datenübertragung, aufweisend
- Bewegen eines zweiten dielektrischen Wellenleiters (10) bei der Datenübertragung gegenüber einem ersten Koppelabschnitt (23) eines ersten dielektrischen Wellenleiters (9) in einer Bewegungsrichtung, wobei ein erster Abstand (d1) des zweiten dielektrischen Wellenleiters (10) zu dem ersten Koppelabschnitt (23) variiert, und wobei ein zweiter Abstand (d2) eines zweiten Koppelabschnitts (25) eines dritten dielektrischen Wellenleiter (12) zu dem zweiten dielektrischen Wellenleiter (10) variiert, wobei
- der erste dielektrische Wellenleiter (9) fest in Bezug auf den dritten dielektrischen Wellenleiter (12) angeordnet ist,
**dadurch gekennzeichnet, dass**
- der erste Koppelabschnitt (23) und der zweite Koppelabschnitt (25) in der Bewegungsrichtung gleich lang sind und senkrecht zu der Bewegungsrichtung einander gegenüber liegen.
